# EUROPEAN PATENT APPLICATION

(11) **EP 2 000 121 A1**
(43) Date of publication of application: **10.12.2008**
(21) Application number: 08155459.4
(22) Date of filing: 30.04.2008
(51) Int. Cl.: A61K 8/19, A61Q 19/00

(54) **Method for demonstrating differences in antioxidant functionality among cosmetic products**

(30) Priority: 05.06.2007 US 758057
(71) Applicant: Unilever PLC, London Greater London EC4P 4BQ (GB); Unilever N.V., 3013 AL Rotterdam (NL)
(72) Inventor: Burger, Allan, Robert, Trumbull, CT 06611 (US)
(74) Representative: Acham, Nicholas Clive

(57) **Abstract**

A method is provided for communicating to consumers through a test protocol chemical functionality of commercially available cosmetics. The method is a proof of the promotional advertising. The steps include comparing two or more different commercially available cosmetic products which advertise antioxidant functionality, contacting each of the products with a test system reactive with antioxidants, and generating a perceivable change in color proportionate to concentration of the antioxidant in the product. The test is preserved in a tangible medium such as a video and then shown to consumers who might have potential purchase interest.

## Description

### BACKGROUND OF THE INVENTION

The invention concerns a method for demonstrating to consumers differences in antioxidant content among competing commercial cosmetic products.

Cosmetic product advertising promises but infrequently delivers the claimed benefit. Cynics aptly refer to this as hope in a bottle.

Most cosmetic advertising claims focus on ingredients which individually constitute less than 0.1% of the formula. Illustrative are the herbal extracts and vitamins. Frequently functionality of an "active" ingredient is highlighted through advertisement. Within the industry the common parlance for these ingredients is "promotional". It is rightly pejorative.

A movement is underway to return integrity to cosmetic product advertising. One avenue is to sell products which actually do contain effective amounts of the ingredient or promised functional activity. But it does cost more to produce. Promotional ingredients ordinarily are the most expensive within the formula. At truly active levels a producer's cost rises significantly, and so will the product selling price. For justification of the high price the consuming public needs to be informed to distinguish products with actives that are not merely present at promotional levels.

Some promotional ingredients even if present at higher levels are not truly active. For instance, esters of certain vitamins need enzymatic deconstruction to release the active structure. Yet when topically applied, the enzymatic process is too slow to render the deconstructed active. Illustrative is vitamin E acetate chemically known as tocopheryl acetate. Many cosmetics advertise the presence of "vitamin E" when in actuality it is the ester version. If topically applied, this ester is not readily antioxidant functional. Enzymatic release is required to form tocopherol. This alcohol is the real vitamin E with antioxidant behavior. A similar situation exists for vitamin C. Often this is delivered as the ester. Topical application does not deliver in real time any ascorbic acid.

### SUMMARY OF THE INVENTION

A method is provided for communicating to consumers through a test protocol chemical functionality of commercially available cosmetics, the method including:
(i) providing a first commercially available cosmetic product that includes an effective amount of an antioxidant in a carrier vehicle;
(ii) providing a second commercially available cosmetic product that includes an ineffective amount of an antioxidant in another carrier vehicle;
(iii) conducting a comparison experiment by subjecting the first and second commercially available cosmetic product to contact with a test system reactive with the antioxidant, the test system generating a perceivable change in color proportionate to concentration of the antioxidant in the product;
(iv) preserving in a tangible medium the comparison experiment; and
(v) exhibiting the preserved medium from step (iv) for viewing by consumers having potential interest in purchase of the first and second commercially available cosmetic products.

### DETAILED DESCRIPTION OF THE INVENTION

Now there has been found a method for educating consumers about which commercially available cosmetic products contain effective amounts of advertised antioxidant. The method is based upon a test system with a color change particularly in the visible spectrum to indicate the antioxidant level. The test system has chemicals reactive with the antioxidant. Upon reaction a color change ensues. Only with effective amounts of antioxidant present will the test system be able to generate the color change. By the term "effective amount of antioxidant" is meant a level sufficient to generate the color change. Ineffective amounts will result in little or no color difference within a test device. Exemplative devices include a test tube, but preferably a water-insoluble substrate such as a cellulosic or plastic strip, charged with interactive color changeable chemicals.

The present invention is not limited to any particular type of cosmetic antioxidant ingredient. Illustrative substances are tocopherol, tocotrienol, ascorbic acid, ferulic acid, green tea, white tea, resveratrol, pomegranate extract and mixtures thereof. Most preferred as a target for the present method is tocopherol and ascorbic acid. The former is available in the isomeric forms of alpha- and gamma- tocopherol. Effective amounts of these materials in sum total or individually may range from 0.5 to 20%, preferably from 0.8 to 5%, and optimally from 1 to 3% by weight of the cosmetic product.

A test system of interactive chemicals particularly suitable for antioxidant detection is that of an electron transfer assay. This is a measure of the ability of the antioxidant species to reduce a chemical reagent. A preferred assay is based on the principle that an antioxidant, acting as an electron donating agent, reduces Mo(VI) to Mo(V). The reagent comprises sodium phosphate and ammonium molybdate in an acidic solution. The molybdate complex, once reduced is blue in color and absorbs visible light at 695 nm. The assay responds strongly to true antioxidants such as vitamin E (tocopherol) and vitamin C (ascorbic acid). It does not respond to derivatives such as vitamin E acetate.

Test systems of the present invention are preferably but not mandatorally conducted in a test tube or with an indicating strip, the latter being impregnated with reagents such as the aforementioned molybdate complex. Impregnated strips are commercially available from Sigma Aldrich Chemical Company under the brand Quantofix. Another effective commercially available strip is sold by EMD Chemicals under the brand EM Quant (No.10023).

Color changes should be observed within 10 to 180 seconds, preferably within 20 to 120 seconds after contact of test system with sample product.

Test systems are not limited to electron transfer assays. A second important class useful for this invention is peroxide based assay. In essence, the assay utilizes a peroxidase for transfer of oxygen from peroxide to an organic redox indictator and forms a blue oxidation product. Assays can be done with a commercially available test strip sold by EMD Chemicals under the brand EM Quant (EMD No. 10011).

An important aspect of this invention is communication with consumers to demonstrate how one commercial product differs from another. More particularly, the communication is to demonstrate via a test system that a first commercially available cosmetic product actually has effective amounts of antioxidant present. This would be compared to a second commercially available cosmetic product with merely promotional amounts of antioxidant present. "Commercially available" for purposes of this invention refers to a cosmetic formula that has been packaged and on sale to end use consumers in a retail establishment.

Primary communication is through video. An assay is videoed wherein samples are shown of the first and second commercially available cosmetic product. Strips of either molybdate complex or hydrogen peroxide strips (e.g. EMD strips) are dipped into each product. After a short period of time, there results on the molybdate complex strips a noticeable difference in color development between the strip dipped into the first product relative to the second product. The peroxide strips after dipping into the product are further developed in dilute hydrogen peroxide solution. Color generated here represents absence of antioxidant. The color changes provide proof to consumers that advertising for the first product is not merely promotional but real in impact.

Video of these tests can be shown on television, through internet websites or other forms of streaming digital images. Alternatively, print versions of the comparison testing could be distributed through billboard advertising, magazines, newspapers, flyers or related media.

Commercially available cosmetic products of the present invention will be compositions that besides antioxidant will also include a cosmetically acceptable carrier. Water is the most preferred carrier. Amounts of water may range from 1 to 99%, preferably from 5 to 90%, more preferably from 35 to 70%, optimally between 40 and 60% by weight of the cosmetic product. Many of the compositions will be water and oil emulsions of the W/O or O/W variety.

Other cosmetically acceptable carriers may include mineral oils, silicone oils, synthetic or natural esters, fatty acids and alcohols and humectants. Amounts of these materials may range from 0.1 to 95%, preferably from 0.5 to 50%, more preferably from 1 to 20% by weight of the cosmetic product.

Silicone oils may be divided into the volatile and non-volatile variety. The term "volatile" as used herein refers to those materials which have a measurable vapor pressure at ambient temperature. Volatile silicone oils are preferably chosen from cyclic or linear polydimethylsiloxanes containing from 3 to 9, preferably from 4 to 5, silicon atoms.

Linear volatile silicone materials generally have viscosities less than 5 centistokes at 25°C while cyclic materials typically have viscosities of less than 10 centistokes.

Non-volatile silicone oils useful as a carrier include polyalkyl siloxanes, polyalkylaryl siloxanes and polyether siloxane copolymers. The essentially non-volatile polyalkyl siloxanes useful herein include, for example, polydimethyl siloxanes with viscosities of from 5 to 100,000 centistokes at 25°C.

Among suitable ester carriers are:
(1) Alkenyl or alkyl esters of fatty acids having 10 to 20 carbon atoms. Examples thereof include isopropyl palmitate, isopropyl isostearate, isononyl isonanonoate, oleyl myristate, oleyl stearate, and oleyl oleate.
(2) Ether-esters such as fatty acid esters of ethoxylated fatty alcohols.
(3) Polyhydric alcohol esters. Ethylene glycol mono- and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol poly-fatty esters, ethoxylated glyceryl monostearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters.
(4) Wax esters such as beeswax, spermaceti, myristyl myristate, stearyl stearate.
(5) Sterols esters, of which soya sterol and cholesterol fatty acid esters are examples thereof.

Fatty acids having from 10 to 30 carbon atoms may also be carriers in the compositions of this invention. Illustrative of this category are pelargonic, lauric, myristic, palmitic, stearic, isostearic, hydroxystearic, oleic, linoleic, ricinoleic, arachidic, behenic and erucic acids.

Humectants of the polyhydric alcohol-type may also be carriers in the cosmetic products (compositions) of this invention. Typical polyhydric alcohols include glycerol (also known as glycerin), polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-butylene glycol, 1,2,6-hexanetriol, ethoxylated glycerol, propoxylated glycerol and mixtures thereof. For best results the humectant is preferably glycerin. The amount of humectant may range anywhere from 0.5 to 30%, preferably between 1 and 15% by weight of the cosmetic product.

Emulsifiers may also be present in cosmetic products of the present invention. Total concentration of the emulsifier may range from 0.1 to 40%, preferably from 1 to 20%, optimally from 1 to 5% by weight of the cosmetic product. The emulsifier may be selected from the group consisting of anionic, nonionic, cationic and amphoteric actives. Particularly preferred nonionic surfactants are those with a C₁₀-C₂₀ fatty alcohol or acid hydrophobe condensed with from 2 to 100 moles of ethylene oxide or propylene oxide per mole of hydrophobe; C₂-C₁₀ alkyl phenols condensed with from 2 to 20 moles of alkylene oxide; mono- and di- fatty acid esters of ethylene glycol; fatty acid monoglyceride; sorbitan, mono- and di-C₈-C₂₀ fatty acids; and polyoxyethylene sorbitan as well as combinations thereof. Alkyl polyglycosides and saccharide fatty amides (e.g. methyl gluconamides) are also suitable nonionic emulsifiers.

Preferred anionic emulsifiers include soap, alkyl ether sulfate and sulfonates, alkyl sulfates and sulfonates, alkylbenzene sulfonates, alkyl and dialkyl sulfosuccinates, C₈-C₂₀ acyl isethionates, C₈-C₂₀ alkyl ether phosphates, alkylethercarboxylates and combinations thereof.

Preservatives can desirably be incorporated into the cosmetic products of this invention to protect against the growth of potentially harmful microorganisms. Suitable traditional preservatives for compositions of this invention are alkyl esters of para-hydroxybenzoic acid. Other preservatives which have more recently come into use include hydantoin derivatives, propionate salts, and a variety of quaternary ammonium compounds. Cosmetic chemists are familiar with appropriate preservatives and routinely choose them to satisfy the preservative challenge test and to provide product stability. Particularly preferred preservatives are iodopropynyl butyl carbamate, phenoxyethanol, methyl paraben, propyl paraben, imidazolidinyl urea, sodium dehydroacetate and benzyl alcohol. The preservatives should be selected having regard for the use of the composition and possible incompatibilities between the preservatives and other ingredients in the emulsion. Preservatives are preferably employed in amounts ranging from 0.01% to 2% by weight of the cosmetic product.

Thickening agents may be included in products of the present invention. Particularly useful are the polysaccharides. Examples include starches, natural/synthetic gums and cellulosics. Representative of the starches are chemically modified starches such as aluminum starch octenylsuccinate. Suitable gums include xanthan, sclerotium, pectin, karaya, arabic, agar, guar, carrageenan, alginate and combinations thereof. Suitable cellulosics include hydroxypropyl cellulose, hydroxypropyl methylcellulose, ethylcellulose and sodium carboxy methylcellulose. Synthetic polymers are still a further class of effective thickening agent. This category includes crosslinked polyacrylates such as the Carbomers and polyacrylamides such as Sepigel® 305.

Amounts of the thickener may range from 0.001 to 5%, preferably from 0.1 to 2%, optimally from 0.2 to 0.5% by weight of the cosmetic product.

Colorants, fragrances and abrasives may also be included in products of the present invention. Each of these substances may range from 0.05 to 5%, preferably between 0.1 and 3% by weight of the cosmetic product.

The following examples will more fully illustrate the embodiments of this invention. All parts, percentages and proportions referred to herein and in the appended claims are by weight unless otherwise indicated.

### EXAMPLE 1

A series of commercially available skin cosmetic products was evaluated with a molybdate antioxidant assay. This assay is based upon the reduction of Mo (VI) to Mo (V) to form a blue/green phosphomolybdate complex. The procedure involved mixing 4 mM ammonium molybdate, 28 mM sodium phosphate and 0.6 M sulfuric acid in water. An extract of each commercially available cosmetic product was made by dissolving one gram of product in 5 ml ethanol. An amount of 0.1 ml of the extract was mixed with 1 ml molybdate complex reagent. The combination was incubated for 1 hour at room temperature. Thereafter it was filtered and evaluated in a spectrophotometer focused on the absorbance 695 nm. Relative absorbance at 695 nm is reported in the table below.

Evident from the table is that the leading commercial face treatment products typically contain low levels of antioxidant. Sample 7, representative of the present invention, is a common base formula but incorporating a very high level (1.25%) of vitamin E (tocopherol). The high antioxidant level was in contrast to the low levels found in the commercial products. Indeed, many of the products had absorbances (antioxidant concentrations) indistinguishable from the assay background (relative background absorbance of about 5%).

A similar result was seen by testing the various products with a Quantofix® test strip. The strips remained yellow or turned only a very light green shade for all the commercial products, except for sample 7. A deep green color resulted from dipping of the test strip in the latter product.

### EXAMPLE 2

EM Quant® Peroxide test strips were utilized in this evaluation. Samples 1, 2, 15, Clinique® Continuous Rescue Antioxidant Moisturizer, and Garnier Nahrologie® were compared against sample 7 (1.25% vitamin E).

A strip was dipped into each of the products and held there for one minute. Excess formulation was then wiped away. The strip was then dipped into 10 ppm of hydrogen peroxide solution for one minute. After about five minutes, a color observation was recorded. Hydrogen peroxide turns the normally colorless strip blue.

Sample 7 completely blocked formation of blue color. All the other products allowed the strip to remain blue indicating that they were all antioxidant inactive. This test appears to be insensitive to sun protection factor (SPF) of any particular product.

**TABLE 1**

| Sample | Commercial Product | Possible Antioxidants | Relative Absorbance |
|---|---|---|---|
| | | (from ingredients label) | (695 nm) |
| 1 | Olay Total Effects 7X | Vitamin E acetate, green tea, Mg ascorbyl phosphate | 4 |
| 2 | Olay Night of Olay Firming Cream | None | 4 |
| 3 | Neutrogena Healthy Skin Anti-Wrinkle | Vitamin E, vitamin E acetate, retinol, BHT, extracts | 20 |
| 4 | Olay Regenerist Eye Lifting Serum | Vitamin E acetate, green tea | 4 |
| 5 | Olay Regenerist Daily Regenerating Serum | Vitamin E acetate, green tea | 4 |
| 6 | Neutrogena Healthy Skin Face Lotion | Vitamin E acetate, retinyl palmitate, ascorbic acid polypeptide | 4 |
| 7 | Experimental facial cream (fortified with 1.25% vitamin E) | Vitamin E (1.25%) | 100 |
| 8 | ROC Age Diminishing Moisturizing Night Cream | Vitamin E acetate, BHT | 6 |
| 9 | ROC Lift & Define Night Cream | Vitamin E acetate | 5 |
| 10 | L'Oreal Wrinkle Decrease Collagen Filler | Vitamin E acetate, extracts | 5 |
| 11 | Olay Regenerist Night | Vitamin E acetate, green tea | 4 |
| 12 | Olay Age Defying Daily Renewal Cream | Vitamin E acetate, salicylic acid | 4 |
| 13 | ROC Retinol Actif Pur Antiwrinkle Day SPF 15 | Vitamin E acetate, BHT, retinol | 18 |
| 14 | L'Oreal Advanced Revitalift Face & Neck Day | None obvious from ingredient list | 14 |
| 15 | L'Oreal Revitalift Complete Multi-Action Cream | Pisum Sativum (pea) extract | 22 |

## Claims

1. A method for communicating to consumers through a test protocol chemical functionality of commercially available cosmetics, the method comprising:
(i) providing a first commercially available cosmetic product comprising an effective amount of an antioxidant in a carrier vehicle;
(ii) providing a second commercially available cosmetic product comprising an ineffective amount of an antioxidant in another carrier vehicle;
(iii) conducting a comparison experiment by subjecting the first and second commercially available cosmetic product to contact with a test system reactive with the antioxidant, the test system generating a perceivable change in color proportionate to concentration of the antioxidant in the product;
(iv) preserving in a tangible medium the comparison experiment; and
(v) exhibiting the preserved medium from step (iv) for viewing by consumers having potential interest in purchase of the first and second commercially available cosmetic products.

2. The method according to claim 1 wherein the test system is based on molybdate (VI) being reduced to molybdate (V) generating a color which absorbs light at 695 nm.

3. The method according to claim 1 wherein the test system is an assay based on peroxide wherein the test system begins colorless and turns blue in the presence of sufficient antioxidant.

4. The method according to claim 1 wherein the test system has indicator chemicals on a strip.

5. The method according to any one of the preceding claims wherein the antioxidant is selected from the group consisting of alpha tocopherol, gamma tocopherol, tocotrienol, ascorbic acid, ferulic acid, green tea, white tea, resveratrol, pomegranate extract and mixtures thereof.

6. The method according to any one of the proceding claims wherein the effective amount of antioxidant ranges from 0.5 to 20% by weight of the cosmetic product.

7. The method according to claim 6 wherein the effective amount of antioxidant ranges from 0.8 to 5% by weight of the cosmetic product.

8. The method according to any one of the preceding claims wherein the tangible medium is selected from the group consisting of television video, internet video, billboard, magazine, newspaper, flyers and combinations thereof.
